# EUROPÄISCHE PATENTANMELDUNG

(11) **EP 3 018 598 A1**
(43) Veröffentlichungstag der Anmeldung: **11.05.2016**
(21) Anmeldenummer: 14191809.4
(22) Anmeldetag: 05.11.2014
(51) Int. Cl.: G06F 19/00

(54) **Auswertungsverfahren für medizinische Daten**

(71) Anmelder: Siemens Aktiengesellschaft, 80333 München (DE)
(72) Erfinder: Dorn, Karlheinz, 90562 Kalchreuth (DE); Goßler, Thomas, 91056 Erlangen (DE); Hewett, Andrew John, Dr., 91058 Erlangen (DE); Ukis, Vladyslav, Dr., 90489 Nürnberg (DE)

(57) **Zusammenfassung**

Mittels einer Anzahl von bildgebenden medizintechnischen Anlagen (1) werden jeweils unter Verwendung eines jeweiligen Messprotokolls (MP) und unter Applizierung einer jeweiligen Dosis (D) Messergebnisse (ME) erfasst. Von den Anlagen (1) oder einem mit der jeweiligen Anlage (1) verbundenen PACS (4) werden automatisch jeweils Datensätze (DS) in eine Cloud (2) eingestellt, die zumindest das jeweils verwendete Messprotokoll (MP), die jeweils applizierte Dosis (D) und in anonymisierter Form aus den erfassten Messergebnissen (ME) jeweils abgeleitete Bilder (B) umfassen. Die Messprotokolle (MP), die applizierten Dosen (D) und die anonymisierten Bilder (B) der Datensätze (DS) sind untereinander referenziert. Den eingestellten Datensätzen (DS) werden zusätzlich der jeweilige Untersuchungstyp (TU) sowie eine Identifikation (IA) der jeweiligen Anlage (1) und/oder eine Identifikation (IT) des Typs der jeweiligen Anlage (1) zugeordnet. Die Cloud (2) nimmt von Nutzern (6) Datenabfragen (RQ) entgegen, die als Suchkriterium zumindest den Untersuchungstyp (TU), die Identifikation (IA) der jeweiligen Anlage (1) und/oder die Identifikation (IT) des Typs der jeweiligen Anlage (1) spezifizieren. Von der Cloud (2) werden entsprechend der jeweiligen Datenabfrage (RQ) die Datensätze (DS) ermittelt und dem jeweiligen Nutzer (6) zur Verfügung gestellt. Die Nutzer (6) spezifizieren in ihren Datenabfragen (RQ) zusätzlich, welche Daten zur Verfügung gestellt werden sollen. Den Nutzern (6) wird von der Cloud (2) die Möglichkeit geboten, ausgehend von den zur Verfügung gestellten Datensätzen (DS) zusätzliche Inhalte dieser Datensätze (DS) abzurufen.

## Beschreibung

Die vorliegende Erfindung betrifft ein Auswertungsverfahren für medizinische Daten.

In Krankenhäusern und anderen medizinischen Einrichtungen wird eine Vielzahl von bildgebenden medizintechnischen Anlagen betrieben. Der Betrieb erfolgt durch Nutzer. Die Nutzer haben oftmals nur eine begrenzte Kenntnis über die Möglichkeiten und Qualitäten der bildgebenden medizintechnischen Anlagen. Sie betreiben die bildgebenden medizintechnischen Anlagen daher in der Regel entsprechend den Vorgaben des Herstellers der jeweiligen bildgebenden medizintechnischen Anlage. In manchen Fällen entwickeln die Nutzer aufgrund ihrer Erfahrung beim Betrieb der jeweiligen bildgebenden medizintechnischen Anlage ein Gefühl dafür, wann und in welchem Umfang sie von den Vorgaben des Herstellers abweichen können. Dies erfordert jedoch in der Regel eine erhebliche Erfahrung. Weiterhin fehlt den Nutzern in der Regel jegliche Möglichkeit, die Qualität und/oder die Optimalität der jeweiligen Betriebsweise der jeweiligen bildgebenden medizintechnischen Anlage einschätzen oder einstufen zu können.

Beim Betrieb der bildgebenden medizintechnischen Anlagen fällt weiterhin eine Vielzahl von Patientendaten an. Insbesondere fallen die mittels der bildgebenden medizintechnischen Anlagen erfassten Bilder an. Diese Bilder können - sofern sie anonymisiert werden - im Prinzip als Lehr- und Vergleichsmaterial verwendet werden. Die Anzahl an in einzelnen Krankenhäusern anfallenden Bildern ist oftmals jedoch als Basis hierfür zu klein.

Die Aufgabe der vorliegenden Erfindung besteht darin, für die Nutzer Möglichkeiten zu schaffen, mittels einer einheitlichen Datenbasis sowohl die Betriebsweise der von ihnen jeweils betriebenen bildgebenden medizintechnischen Anlage einschätzen und einstufen zu können als auch Fallstudien und dergleichen vornehmen zu können.

Die Aufgabe wird durch ein Auswertungsverfahren mit den Merkmalen des Anspruchs 1 gelöst. Vorteilhafte Ausgestaltungen des erfindungsgemäßen Auswertungsverfahrens sind Gegenstand der abhängigen Ansprüche 2 bis 6.

Erfindungsgemäß wird ein Auswertungsverfahren für medizinische Daten geschaffen,
- wobei mittels einer Anzahl von bildgebenden medizintechnischen Anlagen jeweils unter Verwendung eines jeweiligen Messprotokolls und unter Applizierung einer jeweiligen Dosis Messergebnisse erfasst werden,
- wobei von den bildgebenden medizintechnischen Anlagen oder einem mit der jeweiligen bildgebenden medizintechnischen Anlage verbundenen PACS automatisch jeweils Datensätze in eine Cloud eingestellt werden, die zumindest das jeweils verwendete Messprotokoll, die jeweils applizierte Dosis und in anonymisierter Form aus den erfassten Messergebnissen jeweils abgeleitete Bilder umfassen,
- wobei die Messprotokolle, die applizierten Dosen und die anonymisierten Bilder des jeweiligen Datensatzes untereinander referenziert sind,
- wobei den in die Cloud eingestellten Datensätzen zusätzlich der jeweilige Untersuchungstyp sowie eine Identifikation der jeweiligen bildgebenden medizintechnischen Anlage und/oder eine Identifikation des Typs der jeweiligen bildgebenden medizintechnischen Anlage zugeordnet werden,
- wobei die Cloud von Nutzern jeweilige Datenabfragen entgegennimmt,
- wobei die Datenabfragen als Suchkriterium zumindest den Untersuchungstyp und/oder die Identifikation der jeweiligen bildgebenden medizintechnischen Anlage und/oder die Identifikation des Typs der jeweiligen bildgebenden medizintechnischen Anlage spezifizieren,
- wobei von der Cloud entsprechend der jeweiligen Datenabfrage die in die Cloud eingestellten Datensätze ermittelt und dem jeweiligen Nutzer zur Verfügung gestellt werden,
- wobei die Nutzer in ihren Datenabfragen zusätzlich spezifizieren, ob das jeweils verwendete Messprotokoll, die jeweils applizierte Dosis und/oder in anonymisierter Form die jeweiligen Bilder zur Verfügung gestellten werden sollen,
- wobei den Nutzern von der Cloud die Möglichkeit geboten wird, ausgehend von den zur Verfügung gestellten Datensätzen zusätzliche Inhalte dieser Datensätze abzurufen.

Es ist möglich, dass die Datensätze ausschließlich die oben genannten Daten enthalten. Alternativ ist es jedoch möglich, dass die von den bildgebenden medizintechnischen Anlagen oder dem mit der jeweiligen bildgebenden medizintechnischen Anlage verbundenen PACS automatisch in die Cloud eingestellten Datensätze zusätzlich aus den Bildern abgeleitete Daten in anonymisierter Form umfassen. Bei diesen Daten kann es sich beispielsweise um Freitext und/oder eine subjektive qualitative Beurteilung oder Einstufung der Bilder durch den Anwender handeln. Auch diese Daten werden mit den übrigen Daten des jeweiligen Datensatzes verknüpft.

Ebenso ist es möglich, dass die von den bildgebenden medizintechnischen Anlagen oder dem mit der jeweiligen bildgebenden medizintechnischen Anlage verbundenen PACS automatisch in die Cloud eingestellten Datensätze - alternativ oder zusätzlich zu den soeben genannten zusätzlichen Daten - zusätzliche Daten der Bilderfassung umfassen. Bei diesen Daten kann es sich beispielsweise um Parameter der Bilderfassung oder um Charakteristika der jeweiligen bildgebenden medizintechnischen Anlage handeln. Auch diese Daten werden mit den übrigen Daten des jeweiligen Datensatzes verknüpft.

Weiterhin ist es möglich, dass von den bildgebenden medizintechnischen Anlagen oder dem mit der jeweiligen bildgebenden medizintechnischen Anlage verbundenen PACS auch nicht anonymisierte Bilddaten in die Cloud eingestellt werden. Diese Daten werden jedoch in vor unbefugtem Zugriff geschützter Form in die Cloud eingestellt. Weiterhin werden diese Daten - also die nicht anonymisierten Bilddaten - vorzugsweise als eigenständige Datensätze in die Cloud eingestellt, weisen jedoch eine Referenz auf den das verwendete Messprotokoll und die applizierte Dosis enthaltenden Datensatz auf. Dadurch ist zwar, ausgehend von den nicht anonymisierten Bilddaten, der das verwendete Messprotokoll und die applizierte Dosis enthaltende Datensatz auffindbar. Hingegen sind nicht, ausgehend von dem das verwendete Messprotokoll und die applizierte Dosis enthaltenden Datensatz, die nicht anonymisierten Bilddaten auffindbar.

Die bildgebenden medizintechnischen Anlagen können nach Bedarf ausgebildet sein. Beispielsweise können sie jeweils als Röntgenanlage, als CT-Scanner, als C-Bogen-Anlage oder als andere bildgebende medizintechnische Anlage ausgebildet sein.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: ein vernetztes System,
- FIG 2: die Kommunikation mit einer Cloud und
- FIG 3: den Ablauf einer Kommunikation mit der Cloud.

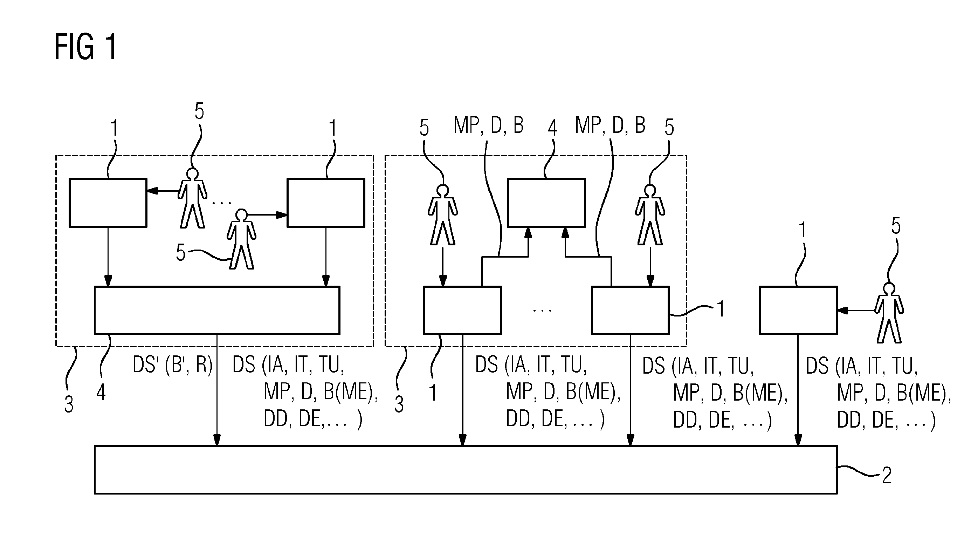

Gemäß FIG 1 ist eine Anzahl von bildgebenden medizintechnischen Anlagen 1 mit einer Cloud 2 verbunden. Die bildgebenden medizintechnischen Anlagen 1 können beispielsweise jeweils als Röntgenanlage, als CT-Scanner oder als C-Bogen-Anlage ausgebildet sein. Alternativ können sie als andere bildgebende medizintechnische Anlage ausgebildet sein, beispielsweise als Magnetresonanzanlage. Die bildgebenden medizintechnischen Anlagen 1 können weltweit verteilt angeordnet sein. Beispielsweise können die bildgebenden medizintechnischen Anlagen 1 innerhalb von Krankenhäusern 3 angeordnet sein und innerhalb des jeweiligen Krankenhauses 3 mit einem jeweiligen PACS 4 (PACS = Picture Archiving and Communication System) verbunden sein.

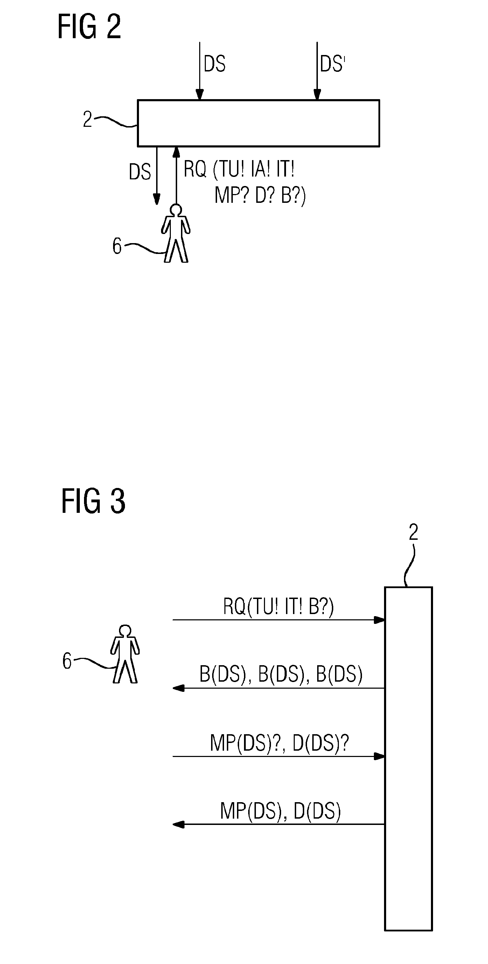

Aufgrund von Vorgaben durch Anwender 5 werden die bildgebenden medizintechnischen Anlagen 1 unter Verwendung eines jeweiligen Messprotokolls MP betrieben. Während der Verwendung des jeweiligen Messprotokolls MP appliziert die jeweilige bildgebende medizintechnische Anlage 1 an einem Patienten (nicht dargestellt) eine jeweilige Dosis D von Strahlung, beispielsweise im Falle einer Magnetresonanzanlage von magnetischer Strahlung oder im Falle einer Röntgenanlage, eines CT-Scanners oder einer C-Bogen-Anlage von ionisierender Strahlung. Aufgrund des Messprotokolls MP werden weiterhin mittels der jeweiligen bildgebenden medizintechnischen Anlage 1 Messergebnisse ME erfasst. Die Messergebnisse ME können bereits selbst direkt Bilder B sein. Alternativ können aus den erfassten Messergebnissen ME Bilder B abgeleitet werden. Die Bilder B und die zur Erfassung der Messergebnisse ME verwendeten Messprotokolle MP werden von der jeweiligen bildgebenden medizintechnischen Anlage 1 - sofern vorhanden - dem jeweiligen PACS 4 zugeführt. Von dem jeweiligen PACS 4 werden sodann automatisch Datensätze DS in die Cloud 2 eingestellt. Die Datensätze DS umfassen - siehe auch FIG 2 - zumindest das jeweils verwendete Messprotokoll MP, die jeweils applizierte Dosis D und die Bilder B. Die Bilder B werden jedoch vor dem Einstellen in die Cloud 2 anonymisiert. Alternativ kann das automatische Einstellen der Datensätze DS auch durch die jeweilige bildgebende medizintechnische Anlage 1 erfolgen. Dies gilt unabhängig davon, ob die jeweilige bildgebende medizintechnische Anlage 1 mit einem PACS 4 verbunden ist oder nicht.

Innerhalb des jeweiligen Datensatzes DS sind das jeweilige Messprotokoll MP die applizierte Dosis D und die anonymisierten Bilder B untereinander referenziert. Es ist also möglich, ausgehend von dem jeweiligen Messprotokoll MP die applizierte Dosis D und die anonymisierten Bilder B des entsprechenden Datensatzes DS aufzufinden. In analoger Weise ist es möglich, ausgehend von der jeweiligen applizierten Dosis D das jeweilige Messprotokoll MP und die anonymisierten Bilder B des entsprechenden Datensatzes DS aufzufinden. In analoger Weise ist es möglich, ausgehend von den anonymisierten Bildern B das Messprotokoll MP und die applizierte Dosis D des entsprechenden Datensatzes DS aufzufinden.

Den Datensätzen DS können weiterhin von der jeweiligen bildgebenden medizintechnischen Anlage 1 oder von dem mit der jeweiligen bildgebenden medizintechnischen Anlage 1 verbundenen PACS 4 zusätzlich in anonymisierter Form Daten DD zugeordnet werden, die aus den Bildern B abgeleitet werden. Auch diese Daten DD werden mit den übrigen Daten des jeweiligen Datensatzes DS verknüpft. Bei diesen Daten DD kann es sich beispielsweise um Freitext oder um eine subjektive qualitative Einstufung der Bilder B handeln. Der Freitext bzw. die Einstufung werden in einem derartigen Fall der jeweiligen bildgebenden medizintechnischen Anlage 1 oder dem entsprechenden PACS 4 vom jeweiligen Anwender 5 vorgegeben. Lediglich das Einstellen dieser Daten DD in die Cloud 2 und das Verknüpfen mit den übrigen Daten MP, D, B des jeweiligen Datensatzes DS erfolgt automatisch.

Weiterhin können den Datensätzen DS zusätzliche Daten DE der Bilderfassung zugeordnet werden. Auch diese Daten DE werden mit den übrigen Daten des jeweiligen Datensatzes DS verknüpft. Bei diesen Daten kann es sich beispielsweise um Parameter der Bilderfassung oder um Charakteristika der jeweiligen bildgebenden medizintechnischen Anlage 1 handeln. Die Zuordnung dieser Daten DE und deren Verknüpfung mit den übrigen Daten des jeweiligen Datensatzes DS kann nach Bedarf von der jeweiligen bildgebenden medizintechnischen Anlage 1 oder von dem mit der jeweiligen bildgebenden medizintechnischen Anlage 1 verbundenen PACS 4 vorgenommen werden. Es ist auch möglich, dass die Zuordnung dieser Daten DE und deren Verknüpfung mit den übrigen Daten MP, D, B und evtl. DD des jeweiligen Datensatzes DS innerhalb der Cloud 2 vorgenommen wird.

Weiterhin werden dem jeweiligen Datensatz DS zusätzlich zu den oben genannten Daten MP, D, B sowie gegebenenfalls DD und/oder DE zusätzlich der jeweilige Untersuchungstyp TU zugeordnet, also beispielsweise ob es sich um eine Untersuchung des Brustkorbes, eines Fußes, einer Hand, eines Oberarms usw. des Patienten handelt. Diese Zuordnung erfolgt durch die jeweilige bildgebende medizintechnische Anlage 1. Weiterhin wird dem jeweiligen Datensatz DS zusätzlich auch eine Identifikation IA der jeweiligen bildgebenden medizintechnischen Anlage 1 zugeordnet. Auch diese Zuordnung erfolgt durch die jeweilige bildgebende medizintechnische Anlage 1. Alternativ oder zusätzlich zum Zuordnen der Identifikation IA kann dem jeweiligen Datensatz DS eine Identifikation IT des Typs der jeweiligen bildgebenden medizintechnischen Anlage 1 zugeordnet werden. Falls die Zuordnung alternativ zum Zuordnen der Identifikation IA erfolgt, wird auch diese Zuordnung von der jeweiligen bildgebenden medizintechnischen Anlage 1 vorgenommen. Falls die Zuordnung zusätzlich zum Zuordnen der Identifikation IA erfolgt, kann diese Zuordnung alternativ von der jeweiligen bildgebenden medizintechnischen Anlage 1, vom zugehörigen PACS 4 oder von der Cloud 2 vorgenommen werden.

Die Cloud 2 nimmt weiterhin gemäß den FIG 2 und 3 von Nutzern 6 - hierbei kann es sich alternativ um die Anwender 5 oder von den Anwendern 5 verschiedene Personen handeln - jeweilige Datenabfragen RQ entgegen. Die Datenabfragen RQ spezifizieren als Suchkriterium zumindest den Untersuchungstyp TU und/oder die Identifikation IA der jeweiligen bildgebenden medizintechnischen Anlage 1 und/oder die Identifikation IT des Typs der jeweiligen bildgebenden medizintechnischen Anlage 1. Dies ist in FIG 2 dadurch angedeutet, dass die entsprechenden Parameter TU, IA, IT mit einem Ausrufezeichen ergänzt sind. Es ist jedoch hinreichend, wenn in der jeweiligen Anfrage RQ nur einer dieser drei Parameter TU, IA, IT vorgegeben wird oder nur zwei dieser drei Parameter TU, IA, IT vorgegeben werden. Weiterhin ist es möglich, auch weitere Parameter als Suchkriterium zu spezifizieren, beispielsweise das Messprotokoll MP oder die applizierte Dosis D. Auch können andere Parameter als Suchkriterium spezifiziert werden, beispielsweise das Datum, zu dem ein bestimmter Datensatz DS erstellt oder in die Cloud 2 eingestellt wurde. Anhand der vorgegebenen Parameter TU, IA, IT werden von der Cloud 2 entsprechend der jeweiligen Datenabfrage RQ die in die Cloud 2 eingestellten Datensätze DS ermittelt und dem jeweiligen Nutzer 6 zur Verfügung gestellt.

In der jeweiligen Datenabfrage RQ spezifizieren die Nutzer 6 zusätzlich, welche Ergebnisse ihnen geliefert werden sollen. Sie spezifizierten also zusätzlich, ob beispielsweise das jeweils verwendete Messprotokoll MP, die jeweils applizierte Dosis D und/oder in anonymisierter Form die jeweiligen Bilder B zur Verfügung gestellt werden sollen. Dies ist in FIG 2 dadurch angedeutet, dass die entsprechenden Parameter MP, D, B mit einem Fragezeichen ergänzt sind. Sofern der Untersuchungstyp TU, die Identifikation IA der jeweiligen bildgebenden medizintechnischen Anlage 1 und die Identifikation IT des Typs der jeweiligen bildgebenden medizintechnischen Anlage 1 nicht bereits im Rahmen der jeweiligen Datenabfrage RQ als Suchkriterium vorgegeben werden, können auch diese Parameter TU, IA, IT als zu liefernde Ergebnisse spezifiziert werden.

Ausgehend von den zur Verfügung gestellten Datensätzen DS (bzw. deren abgefragten Elementen) wird dem jeweiligen Nutzer 6 weiterhin entsprechend FIG 3 von der Cloud 2 die Möglichkeit geboten, zusätzliche Inhalte dieser Datensätze DS abzurufen. Beispielsweise kann der jeweiligen Nutzer 6, nachdem er - wie in FIG 3 rein beispielhaft dargestellt - zunächst nur den jeweiligen Untersuchungstyp TU als Suchkriterium spezifiziert hat und als zu liefernde Daten die zugehörigen Bilder B der betreffenden Datensätze DS spezifiziert hat, einen oder mehrere der übermittelten Datensätze DS auswählen und bezüglich der ausgewählten Datensätze DS deren Messprotokolle MP und die zugehörigen Dosen D abrufen.

Aufgrund der in die Cloud 2 eingestellten Datensätze DS ist eine Vielzahl von Auswertungen möglich. Hierzu einige Beispiele:
Wenn ein Nutzer 6 beispielsweise erfahren möchte, wieviel Dosis D für eine bestimmte Untersuchung benötigt wird, spezifiziert er als Suchkriterium den Untersuchungstyp TU und den Typ IT der ihm zur Verfügung stehenden bildgebenden medizintechnischen Anlage 1. Als zu liefernde Daten spezifiziert er die Bilder B der entsprechenden Datensätze DS. Für Bilder B, die der Nutzer 6 als gut bzw. hinreichend gut erachtet, fragt er zusätzlich das verwendete Messprotokoll MP und/oder die applizierte Dosis D ab. Dadurch kann der Nutzer 6 eine Optimierung des Betriebs der ihm zur Verfügung stehenden bildgebenden medizintechnischen Anlage 1 vornehmen.

Wenn ein Nutzer 6 wissen möchte, welche Ergebnisse mit einem bestimmten Messprotokoll MP erhalten werden, spezifiziert er als Suchkriterium den Untersuchungstyp TU und das Messprotokoll MP. Zusätzlich kann er als Suchkriterium gegebenenfalls den Typ IT der ihm zur Verfügung stehenden bildgebenden medizintechnischen Anlage 1 spezifizieren. Als zu liefernde Daten spezifiziert der Nutzer 6 in diesem Fall die Bilder B. Bei guten Bildern B fragt er weiterhin die applizierte Dosis D ab. Auch dadurch kann der Nutzer 6 eine Optimierung des Betriebs der ihm zur Verfügung stehenden bildgebenden medizintechnischen Anlage 1 vornehmen.

Wenn der Nutzer 6 eine Einschätzung der von ihm beim Betrieb der bildgebenden medizintechnischen Anlage 1 ergriffenen Vorgehensweise vornehmen möchte, spezifiziert er als Suchkriterium den Untersuchungstyp TU und eventuell zusätzlich den Typ IT der ihm zur Verfügung stehenden bildgebenden medizintechnischen Anlage 1. Als zu liefernde Daten spezifiziert er die Bilder B und die applizierte Dosis D. Weiterhin spezifiziert er, dass die Daten mit aufsteigender oder absteigender applizierter Dosis D ausgegeben werden.

Zusätzlich ist es entsprechend der Darstellung in FIG 1 möglich, dass von den bildgebenden medizintechnischen Anlagen 1 oder dem mit der jeweiligen bildgebenden medizintechnischen Anlage 1 verbundenen PACS 4 auch nicht anonymisierte Bilddaten B' in die Cloud 2 eingestellt werden. Dieses Einstellen erfolgt jedoch nur dann, wenn es zuvor vom jeweiligen Anwender 5 autorisiert wird. Weiterhin erfolgt das Einstellen in einer Form, in der die Bilddaten B' vor unbefugtem Zugriff geschützt sind. Beispielsweise können die Daten verschlüsselt und/oder durch ein Passwort geschützt sein. Dadurch ist es möglich, über die Cloud 2 auch sensitive Bilddaten B' zwischen verschiedenen Orten und damit verschiedenen Anwendern 5 und Nutzern 6 auszutauschen.

In der Regel werden die nicht anonymisierten Bilddaten B' gemäß FIG 2 als eigenständige Datensätze DS' in die Cloud 2 eingestellt. Sie enthalten jedoch in der Regel eine Referenz R auf den Datensatz DS, der das verwendete Messprotokoll MP und die applizierte Dosis D enthält. Somit kann zwar, ausgehend von den nicht anonymisierten Bilddaten B', der Datensatz DS aufgefunden werden, der das verwendete Messprotokoll MP und die applizierte Dosis D enthält. Hingegen können nicht umgekehrt, ausgehend von dem Datensatz DS, der das verwendete Messprotokoll MP und die applizierte Dosis D enthält, die nicht anonymisierten Bilddaten B' aufgefunden werden.

Zusammengefasst betrifft die vorliegende Erfindung somit folgenden Sachverhalt:
Mittels einer Anzahl von bildgebenden medizintechnischen Anlagen 1 werden jeweils unter Verwendung eines jeweiligen Messprotokolls MP und unter Applizierung einer jeweiligen Dosis D Messergebnisse ME erfasst. Von den Anlagen 1 oder einem mit der jeweiligen Anlage 1 verbundenen PACS 4 werden automatisch jeweils Datensätze DS in eine Cloud 2 eingestellt, die zumindest das jeweils verwendete Messprotokoll MP, die jeweils applizierte Dosis D und in anonymisierter Form aus den erfassten Messergebnissen ME abgeleitete Bilder B umfassen. Die Messprotokolle MP, die applizierten Dosen D und die anonymisierten Bilder B der Datensätze DS sind untereinander referenziert. Den eingestellten Datensätzen DS werden zusätzlich der jeweilige Untersuchungstyp TU sowie eine Identifikation IA der jeweiligen Anlage 1 und/oder eine Identifikation IT des Typs der jeweiligen Anlage 1 zugeordnet. Die Cloud 2 nimmt von Nutzern 6 Datenabfragen RQ entgegen, die als Suchkriterium zumindest den Untersuchungstyp TU, die Identifikation IA der jeweiligen Anlage 1 und/oder die Identifikation IT des Typs der jeweiligen Anlage 1 spezifizieren. Von der Cloud 2 werden entsprechend der jeweiligen Datenabfrage RQ die Datensätze DS ermittelt und dem jeweiligen Nutzer 6 zur Verfügung gestellt. Die Nutzer 6 spezifizieren in ihren Datenabfragen RQ zusätzlich, welche Daten zur Verfügung gestellt werden sollen. Den Nutzern 6 wird von der Cloud 2 die Möglichkeit geboten, ausgehend von den zur Verfügung gestellten Datensätzen DS zusätzliche Inhalte dieser Datensätze DS abzurufen.

Die vorliegende Erfindung weist viele Vorteile auf. So wird insbesondere eine umfassende, einheitliche Datenbasis geschaffen, welche weltweit einheitlich ausgewertet werden kann. Darüber hinaus ist auch ein individualisierter Austausch vertraulicher Patientendaten möglich. Aufgrund des automatisierten Einstellens der Datensätze DS wird weiterhin die Cloud 2 automatisch kontinuierlich erweitert, die Datenbasis also verbreitert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: bildgebende medizintechnische Anlagen (bgmtA)
- 2: Cloud
- 3: Krankenhäuser
- 4: PACS
- 5: Anwender
- 6: Nutzer

- B: Bilder
- B': Bilddaten
- D: Dosis
- DD: zusätzliche Daten
- DE: zusätzliche Daten
- DS, DS': Datensätze
- IA: Identifikation der bgmtA
- IT: Identifikation des Typs der bgmtA
- ME: Messergebnisse
- MP: Messprotokoll
- R: Referenz
- RQ: Datenabfragen
- TU: Untersuchungstyp

## Patentansprüche

1. Auswertungsverfahren für medizinische Daten,
- wobei mittels einer Anzahl von bildgebenden medizintechnischen Anlagen (1) jeweils unter Verwendung eines jeweiligen Messprotokolls (MP) und unter Applizierung einer jeweiligen Dosis (D) Messergebnisse (ME) erfasst werden,
- wobei von den bildgebenden medizintechnischen Anlagen (1) oder einem mit der jeweiligen bildgebenden medizintechnischen Anlage (1) verbundenen PACS (4) automatisch jeweils Datensätze (DS) in eine Cloud (2) eingestellt werden, die zumindest das jeweils verwendete Messprotokoll (MP), die jeweils applizierte Dosis (D) und in anonymisierter Form aus den erfassten Messergebnissen (ME) jeweils abgeleitete Bilder (B) umfassen,
- wobei die Messprotokolle (MP), die applizierten Dosen (D) und die anonymisierten Bilder (B) des jeweiligen Datensatzes (DS) untereinander referenziert sind,
- wobei den in die Cloud (2) eingestellten Datensätzen (DS) zusätzlich der jeweilige Untersuchungstyp (TU) sowie eine Identifikation (IA) der jeweiligen bildgebenden medizintechnischen Anlage (1) und/oder eine Identifikation (IT) des Typs der jeweiligen bildgebenden medizintechnischen Anlage (1) zugeordnet werden,
- wobei die Cloud (2) von Nutzern (6) jeweilige Datenabfragen (RQ) entgegennimmt,
- wobei die Datenabfragen (RQ) als Suchkriterium zumindest den Untersuchungstyp (TU) und/oder die Identifikation (IA) der jeweiligen bildgebenden medizintechnischen Anlage (1) und/oder die Identifikation (IT) des Typs der jeweiligen bildgebenden medizintechnischen Anlage (1) spezifizieren,
- wobei von der Cloud (2) entsprechend der jeweiligen Datenabfrage (RQ) die in die Cloud (2) eingestellten Datensätze (DS) ermittelt und dem jeweiligen Nutzer (6) zur Verfügung gestellt werden,
- wobei die Nutzer (6) in ihren Datenabfragen (RQ) zusätzlich spezifizieren, ob das jeweils verwendete Messprotokoll (MP), die jeweils applizierte Dosis (D) und/oder in anonymisierter Form die jeweiligen Bilder (B) zur Verfügung gestellt werden sollen,
- wobei den Nutzern (6) von der Cloud (2) die Möglichkeit geboten wird, ausgehend von den zur Verfügung gestellten Datensätzen (DS) zusätzliche Inhalte dieser Datensätze (DS) abzurufen.

2. Auswertungsverfahren nach Anspruch 1,
**dadurch gekennzeichnet,**
**dass** die von den bildgebenden medizintechnischen Anlagen (1) oder dem mit der jeweiligen bildgebenden medizintechnischen Anlage (1) verbundenen PACS (4) automatisch in die Cloud (2) eingestellten Datensätze (DS) zusätzlich aus den Bildern (B) abgeleitete Daten (DD) in anonymisierter Form umfassen und dass auch diese Daten (DD) mit den übrigen Daten des jeweiligen Datensatzes (DS) verknüpft werden.

3. Auswertungsverfahren nach Anspruch 1 oder 2,
**dadurch gekennzeichnet,**
**dass** die von den bildgebenden medizintechnischen Anlagen (1) oder dem mit der jeweiligen bildgebenden medizintechnischen Anlage (1) verbundenen PACS (4) automatisch in die Cloud (2) eingestellten Datensätze (DS) zusätzliche Daten (DE) der Bilderfassung umfassen und dass auch diese Daten (DE) mit den übrigen Daten des jeweiligen Datensatzes (DS) verknüpft werden.

4. Auswertungsverfahren nach Anspruch 1, 2 oder 3,
**dadurch gekennzeichnet,**
**dass** von den bildgebenden medizintechnischen Anlagen (1) oder dem mit der jeweiligen bildgebenden medizintechnischen Anlage (1) verbundenen PACS (4) in vor unbefugtem Zugriff geschützter Form auch nicht anonymisierte Bilddaten (B') in die Cloud (2) eingestellt werden.

5. Auswertungsverfahren nach Anspruch 4,
**dadurch gekennzeichnet,**
**dass** die nicht anonymisierten Bilddaten (B') als eigenständige Datensätze (DS') in die Cloud (2) eingestellt werden, jedoch eine Referenz (R) auf den das verwendete Messprotokoll (MP) und die applizierte Dosis (D) enthaltenden Datensatz (DS) aufweisen.

6. Auswertungsverfahren nach einem der obigen Ansprüche,
**dadurch gekennzeichnet,**
**dass** die bildgebenden medizintechnischen Anlagen (1) jeweils als Röntgenanlage, als CT-Scanner, als C-Bogenanlage oder als andere bildgebende medizintechnische Anlage ausgebildet sind.
